# EUROPEAN PATENT APPLICATION

(11) **EP 2 520 246 A1**
(43) Date of publication of application: **07.11.2012**
(21) Application number: 11734521.5
(22) Date of filing: 21.01.2011
(51) Int. Cl.: A61C 7/08, A61C 19/06

(54) **BASE MATERIAL FORMING THERAPEUTIC DENTAL MOUTHPIECE AND PREFORMING METHOD**

(30) Priority: 25.01.2010 JP 2010013183
(71) Applicant: Panasonic Corporation, Kadoma-shi Osaka 571-8501 (JP)
(72) Inventor: SAKIMURA, Takumi, 2-1-61 Shiromi, Chuo-ku Osaka 540-6207 (JP); FUKUSHIMA, Shogo, 2-1-61 Shiromi, Chuo-ku Osaka 540-6207 (JP)
(74) Representative: Müller-Boré & Partner Patentanwälte
(86) International application number: PCT/JP2011/000304
(87) International publication number: WO 2011/089914

(57) **Abstract**

A therapeutic dental mouthpiece-forming substrate 2(A) includes: a flexible board 6 on which an electrical component 5(A) or 5(B) used for dental arch treatment is mounted; and a thermosoftening resin sheet 1 in which the flexible board 6 is embedded in an airtight or watertight state.

## Description

### Technical Field

The present invention relates to a substrate for forming a therapeutic dental mouthpiece and a preforming method thereof.

### Background Art

Mouthpieces are becoming more widespread not only for protecting teeth (as a mouth guard) during sport but also for use in dental treatment. However, dental arches differ in size and shape among different users, and therefore the mouthpiece must be formed along the dental arch of the user.

To form this type of mouthpiece, either a method in which the user forms the mouthpiece from his/her own dental arch or a method in which a dentist or a dental technician forms the mouthpiece from a dental model extracted from the user is typically employed.

In the former method, a commercially available steric molded body (a semi-finished product) preformed in a steric shape from resin so as to take an arc shape (an arch shape) that follows a curve of the dental arch when seen from above and a U shape that fits over the dental arch when seen from the side is used. The user can form the steric molded body into a finished product by attaching the molded body to his/her own dental arch after softening it in warm water (between 70°C and 80°C) and then closing his/her upper and lower dental arches, holding the dental arch with a finger, and so on.

The operation employed in this method is extremely simple, and therefore the method is widely used for sport mouthpieces. However, disadvantages thereof include poor alignment (a poor fit) between the mouthpiece and the dental arch, the need to take care during forming due to the high temperature, and so on.

In the latter method, a commercially available disc-shaped or quadrangular resin sheet is used. The dentist or the like can form the resin sheet into a finished product by setting the resin sheet on a commercially available forming apparatus, where the resin sheet is softened, suctioned, and the like so as to be adhered tightly to the dental model of the user.

The mouthpiece obtained in this method is favorable in terms of the alignment with the dental arch, but specialist operations are required to manufacture the dental model and form the mouthpiece.

Incidentally, a mouthpiece for performing whitening treatment on a dental arch using light of a specific wavelength (see Patent Document 1), a mouthpiece that promotes an orthodontic treatment effect by applying vibration to a dental arch (see Patent Document 2), and so on have been proposed in recent years.

With the mouthpiece used for whitening treatment, electrical components such as a light emitting element, a control element thereof, a power supply battery, and a power supply switch must be incorporated into the mouthpiece. Further, with the mouthpiece used for orthodontic treatment, electrical components such as a vibrator, a control element thereof, a power supply battery, and a power supply switch must be incorporated into the mouthpiece.

To obtain a sufficient therapeutic effect when this type of electrical component is incorporated into the mouthpiece, measures must be taken to ensure that the electrical component is disposed accurately in a predetermined treatment position, and that the mouthpiece can be attached with stability so as to align with (fit) the dental arch.

Further, since the electrical components are inserted into the oral cavity together with the mouthpiece, it is necessary in terms of safety to ensure that the electrical components are embedded in the mouthpiece in an airtight or watertight state.

It is difficult for a user, a dentist, or the like who is not accustomed to handling such electrical components to incorporate the electrical components him/herself, and therefore mouthpieces having embedded electrical components may be formed by a manufacturer or a vendor who is accustomed to handling the electrical components.

Patent Document 1: Japanese Patent Application Publication No. 2005-342072
Patent Document 2: Japanese Patent Application Publication No. 2007-260158

In order to obtain the dental model of the user, however, the manufacturer or vendor must construct a network with a vast number of dentists and the like, which involves great labor and cost. Further, when the user forms the mouthpiece him/herself, the user must form the mouthpiece accurately after selecting appropriate electrical components corresponding to the application and the case. Hence, neither method can be considered realistic.

### Summary of the Invention

The present invention has been designed to solve the problems described above, and an object thereof is to provide a therapeutic dental mouthpiece-forming substrate and a preforming method thereof, with which a mouthpiece including an embedded electrical component can be formed safely and at low cost.

A therapeutic dental mouthpiece-forming substrate according to the present invention includes: a flexible board on which an electrical component used for dental arch treatment is mounted; and a thermosoftening resin sheet in which the flexible board is embedded in an airtight or watertight state.

### Brief Description of the Drawings

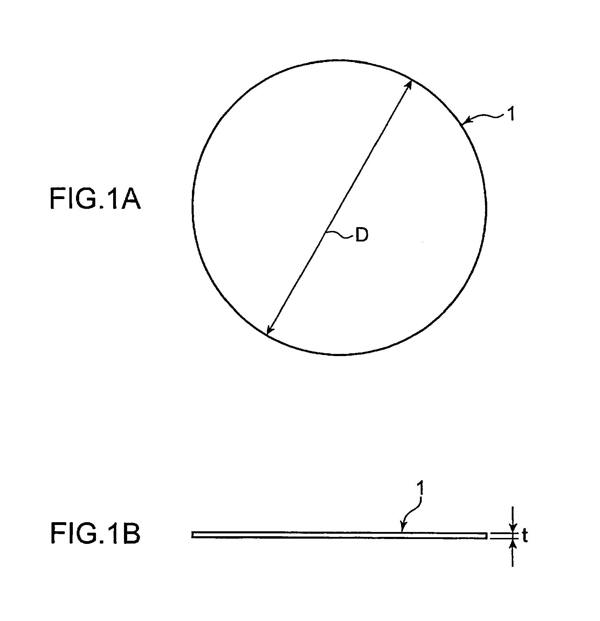

Figs. 1A and 1B show an example of a commercially available thermosoftening resin sheet used as a mouthpiece, wherein Fig. 1A is a plan view and Fig. 1B is a side view.

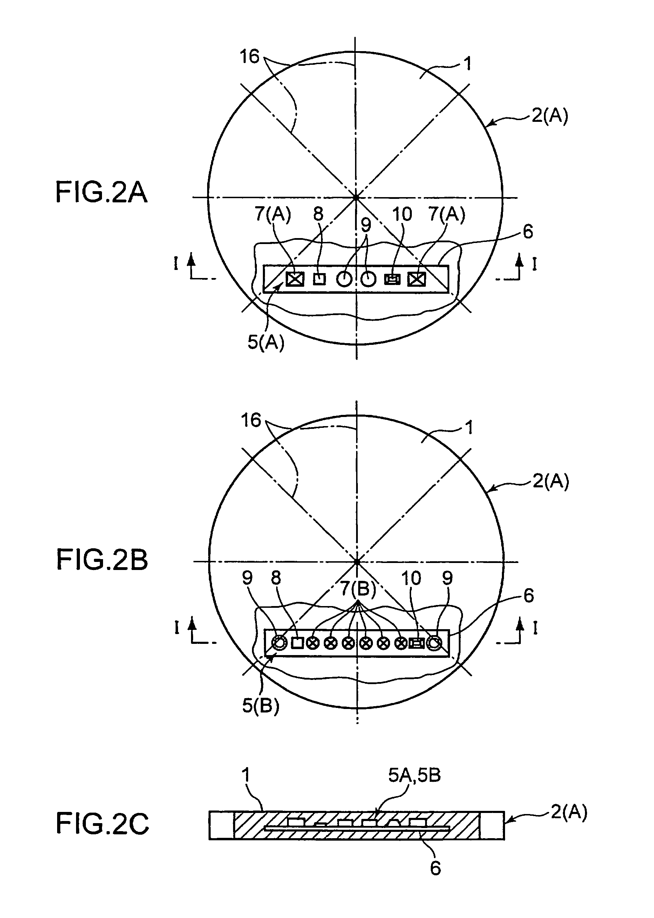

Figs. 2A and 2B show a mouthpiece-forming substrate according to a first embodiment of the present invention, wherein Fig. 2A is a partially cutaway plan view showing main parts of the mouthpiece-forming substrate including an embedded flexible board on which an electrical component used in orthodontic treatment of a dental arch is mounted, Fig. 2B is a partially cutaway plan view showing main parts of the mouthpiece-forming substrate including an embedded flexible board on which an electrical component used in whitening treatment of a dental arch is mounted, and Fig. 2C is a sectional view corresponding to an I-I line in each of Figs. 2A and 2B.

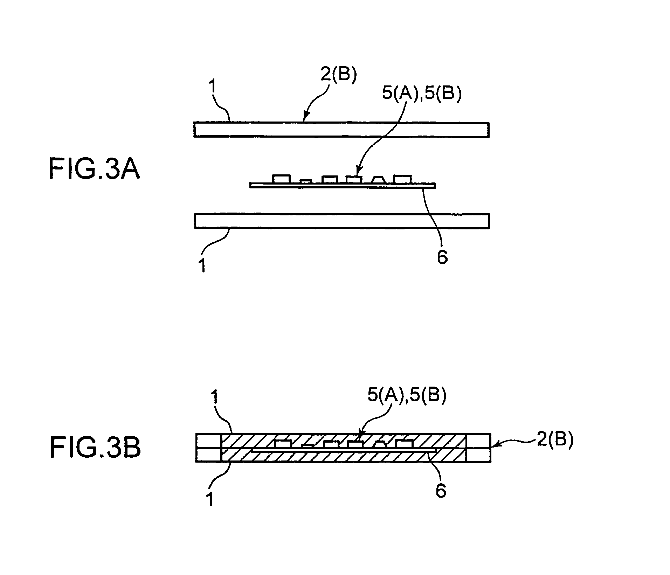

Figs. 3A and 3B show a mouthpiece-forming substrate according to a second embodiment of the present invention, wherein Fig. 3A is an exploded side view showing the mouthpiece-forming substrate before embedding a flexible board on which an electrical component is mounted, and Fig. 3B is a sectional view corresponding to an I-I line in each of Figs. 2A and 2B.

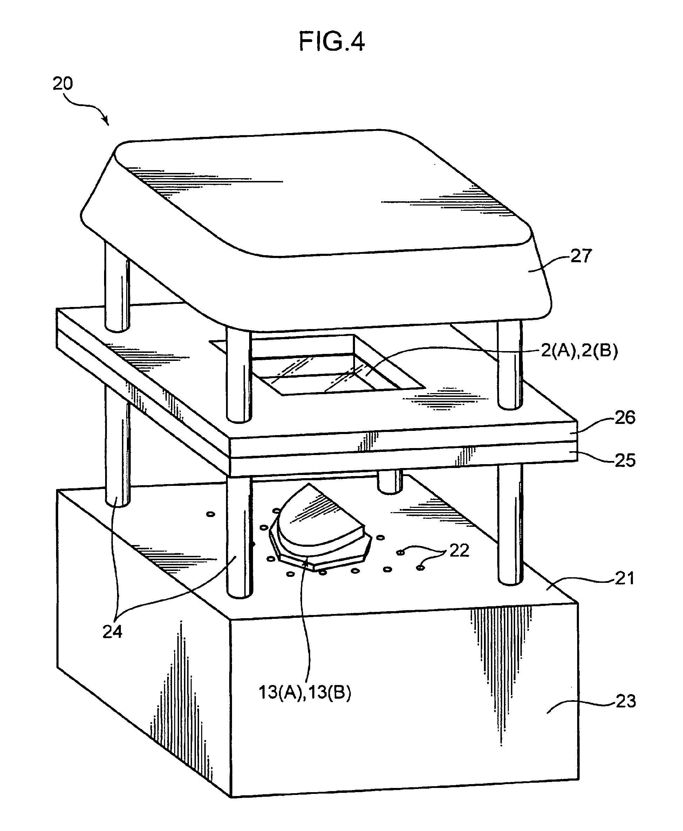

Fig. 4 is a perspective view showing a forming apparatus for preforming the mouthpiece-forming substrate according to the present invention into a steric shape.

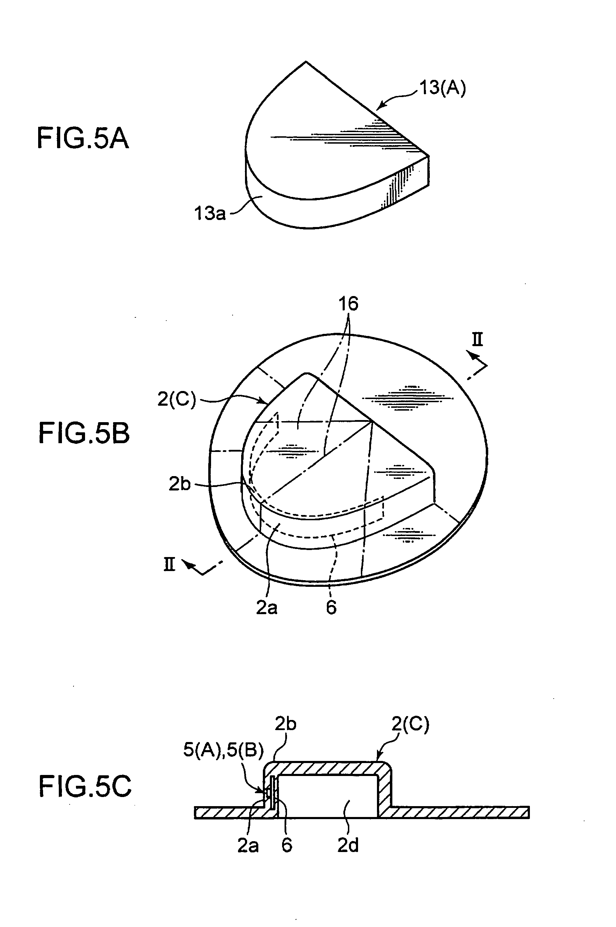

Figs. 5A and 5B show an outline of an operation for preforming the mouthpiece-forming substrate into a steric shape using a general purpose dental model according to a first example of the present invention, wherein Fig. 5A is a perspective view of the general purpose dental model, Fig. 5B is a perspective view of a mouthpiece-forming substrate preformed into a steric shape, and Fig. 5C is a sectional view corresponding to a II-II line in Fig. 5B.

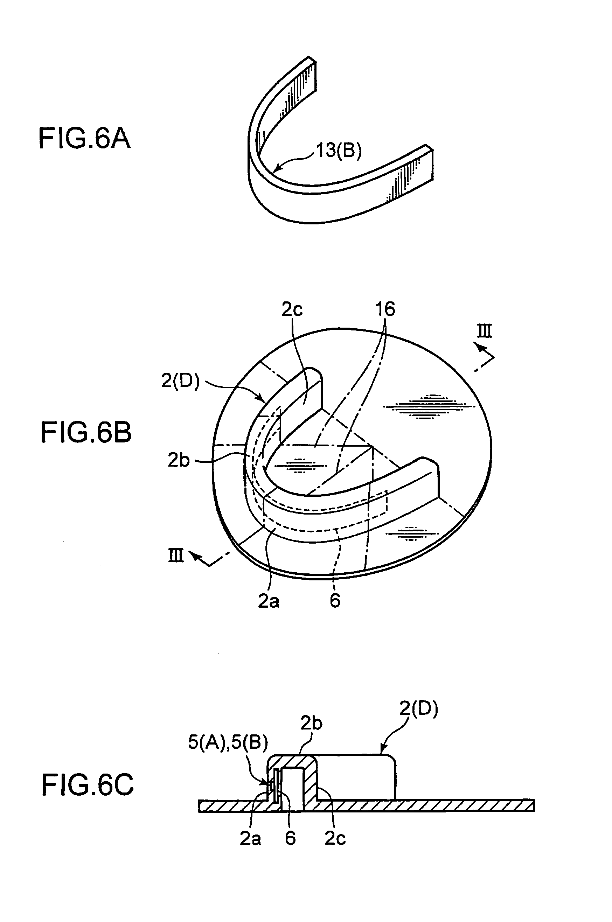

Figs. 6A and 6B show an outline of an operation for preforming the mouthpiece-forming substrate into a steric shape using a general purpose dental model according to a second example of the present invention, wherein Fig. 6A is a perspective view of the general purpose dental model, Fig. 6B is a perspective view of a mouthpiece-forming substrate preformed into a steric shape, and Fig. 6C is a sectional view corresponding to a III-III line in Fig. 6B.

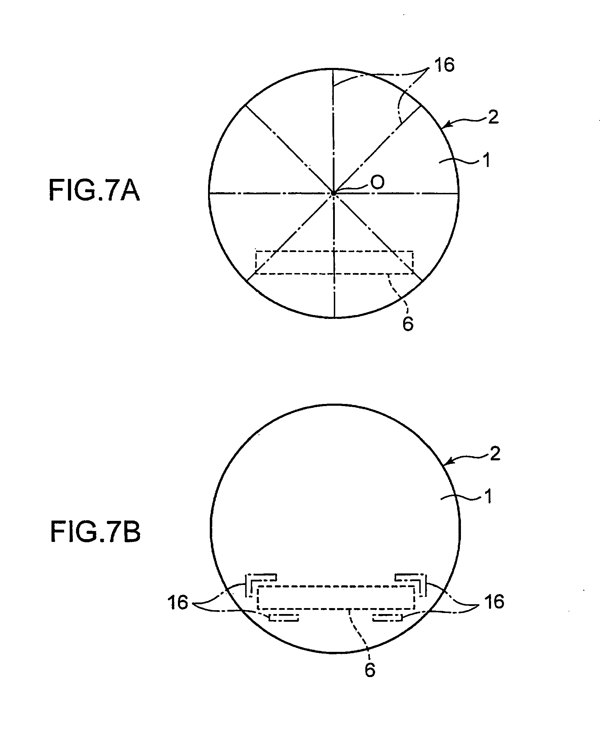

Figs. 7A and 7B are plan views respectively showing a mouthpiece-forming substrate in which positioning marks are provided on the mouthpiece-forming substrate.

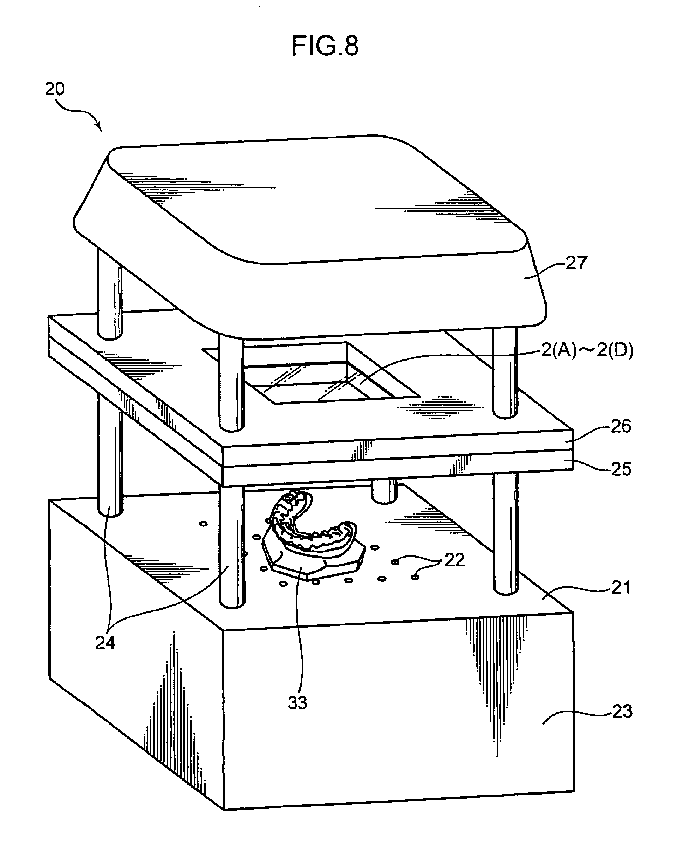

Fig. 8 is a perspective view showing a forming apparatus for forming a mouthpiece using a mouthpiece-forming substrate preformed into a steric shape.

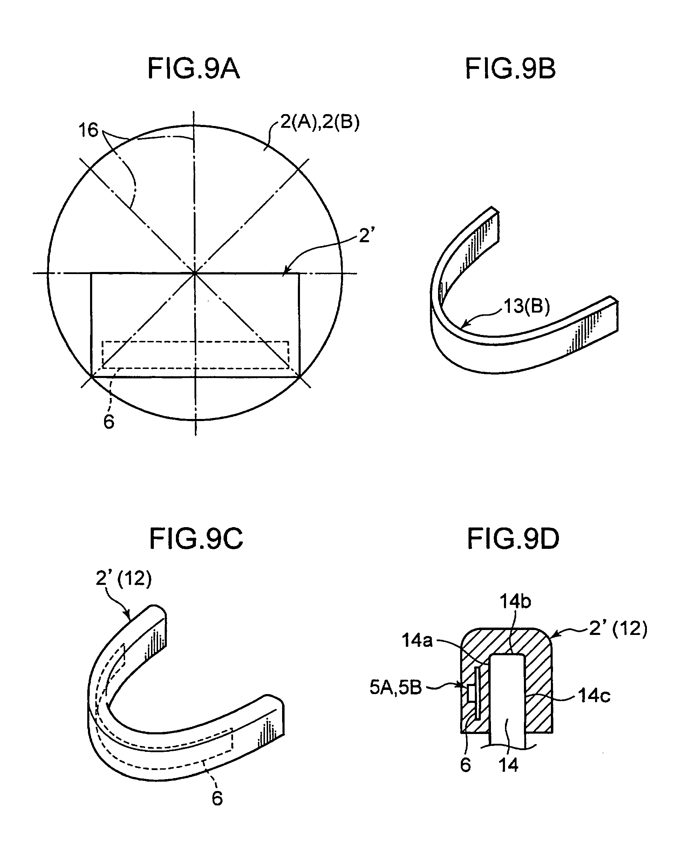

Figs. 9A and 9B show an outline of an operation for preforming a substrate piece into a steric shape using a mouthpiece-forming sheet-form substrate according to the present invention, wherein Fig. 9A is a plan view of the mouthpiece-forming sheet-form substrate, Fig. 9B is a perspective view of a general purpose dental model, Fig. 9C is a perspective view of the substrate piece preformed into a steric shape, and Fig. 9D is a sectional view of Fig. 9C.

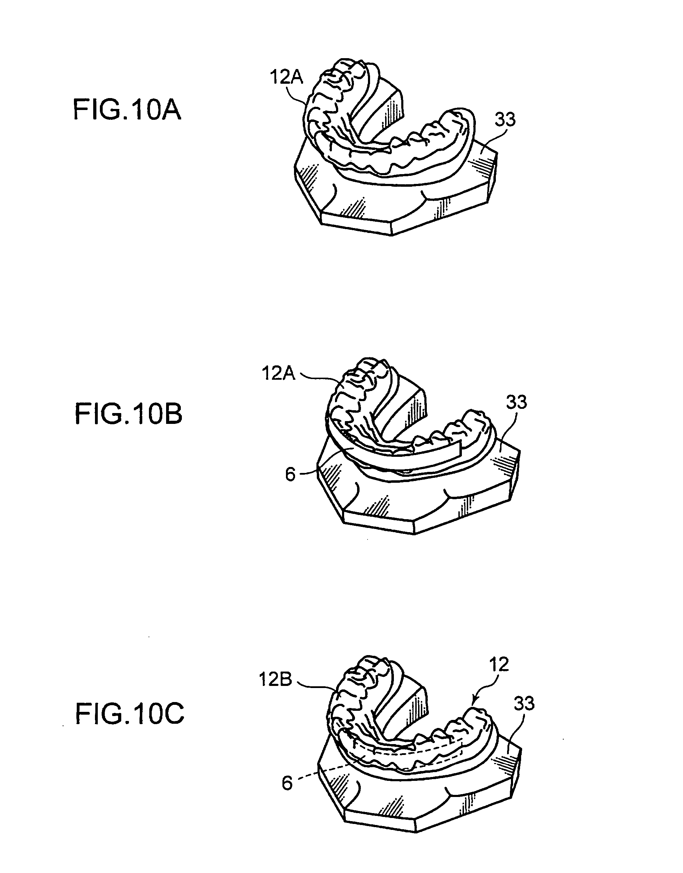

Figs. 10A and 10B are a process diagram showing a process for forming a mouthpiece using two resin sheets, wherein Fig. 10A shows a state in which a first resin sheet has been formed into an inside piece by heating and suction, Fig. 10B shows a state in which a flexible board has been attached to the inside piece, and Fig. 10C shows a state in which an outside piece has been formed by heating and suctioning a second resin sheet.

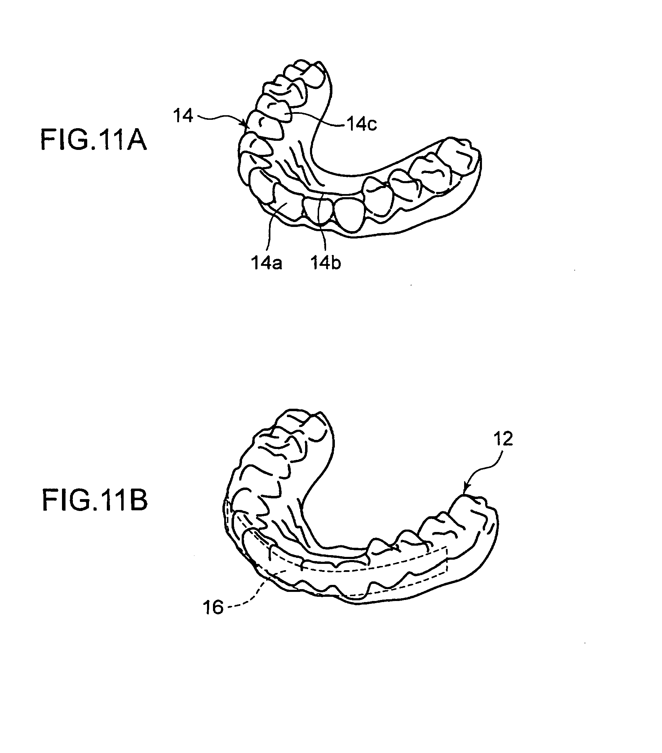

Fig. 11A is a perspective view of a dental arch, and Fig. 11B is a perspective view of a mouthpiece.

### Description of Embodiments

Embodiments of the present invention will be described in detail below with reference to the drawings.

Figs. 1A and 1B show an example of a commercially available thermosoftening resin sheet 1 used as a mouthpiece, wherein Fig. 1A is a plan view and Fig. 1B is a side view.

A size of the commercially available disc-shaped thermosoftening resin sheet 1 used as a mouthpiece is typically set such that an outer diameter D is approximately 125 mm and a thickness t is approximately 1 to 6 mm. Note that a quadrangular thermosoftening resin sheet of a similar size is also commercially available. When two thermosoftening resin sheets 1 are overlapped for use, as in a second embodiment to be described below, the thickness t of one sheet is preferably set at approximately 1.5 mm.

A material (a polymeric material such as a thermoplastic resin, for example) that is used widely as a mouthpiece (or mouth guard) material and guaranteed to be safe in terms of hygiene is used as a material of the thermosoftening resin sheet 1. For example, a sheet of EVA (ethylene vinyl acetate copolymer), which is a polymeric material, can be used favorably. By forming the EVA in a manner to be described below, allergic reactions on the teeth and gums can be suppressed. The material of the thermosoftening resin sheet 1 is not limited to the aforesaid EVA sheet, but with an EVA sheet, electrical insulation can be secured such that the EVA sheet also functions as a heat insulator. Hence, an EVA sheet is preferable. Particularly in the second embodiment to be described below, when an EVA sheet is used as a first thermosoftening resin sheet 1, an inside piece 12A (see Fig. 10A) that directly contacts a surface (a front surface) of a dental arch can be made flexible. As a result, a mitigating effect acts on a high speed component of mechanical vibration transmitted from a vibrator 7(A) (see Fig. 2A) to a part corresponding to a correction subject tooth, and therefore damage to the part corresponding to the correction subject tooth can be forestalled.

Figs. 2A to 2C show a therapeutic dental mouthpiece-forming sheet-form substrate 2(A) a according to a first embodiment. Fig. 2A is a partially cutaway plan view showing main parts of the therapeutic dental mouthpiece-forming sheet-form substrate 2(A) including an embedded flexible board 6 on which an electrical component 5(A) used in orthodontic treatment of a dental arch is mounted. Fig. 2B is a partially cutaway plan view showing main parts of the therapeutic dental mouthpiece-forming sheet-form substrate 2(A) including an embedded flexible board 6 on which an electrical component 5(B) used in whitening treatment of the dental arch is mounted. Fig. 2C is a sectional view corresponding to an I-I line in each of Figs. 2A and 2B.

The flexible board 6 is a thin, flexible printed wiring board. In the flexible board 6 on which the electrical component 5(A) used in orthodontic treatment of the dental arch is mounted, shown in Fig. 2A, a vibrator (oscillation motor) 7(A) used in orthodontic treatment of a dental arch is mounted as the electrical component 5(A). Further, a control element 8 of the vibrator 7(A), a power supply battery 9 such as a button battery, and a push type power supply switch 10 are mounted in appropriate locations. Note that the vibrator 7(A) is mounted in the site of the correction subject tooth.

Further, in the flexible board 6 on which the electrical component 5(B) used in whitening treatment of the dental arch is mounted, shown in Fig. 2B, a light emitting element 7(B) used in whitening treatment of a dental arch is mounted as the electrical component 5(B). Further, a control element 8 of the light emitting element 7(B), a power supply battery 9 such as a button battery, and a power supply switch 10 are mounted in appropriate locations. Note that the light emitting element 7(B) is mounted in a site of a whitening subject tooth.

A mouthpiece 12 to be described below (see Fig. 11B) may be formed by mounting only the vibrator 7(A) or the light emitting element 7(B) on the flexible board 6. A lead (a cord) thereof may then be drawn to the exterior of an oral cavity and electrically connected to the control element 8, the power supply battery 9, and the power supply switch 10 on the exterior of the oral cavity.

Further, the flexible board 6 may be mounted with a switching element instead of the push type power supply switch 10 and with a power feeding coil and a chargeable battery instead of the power supply battery 9 such as a button battery. In so doing, the switching element can be used to perform a switching operation on the vibrator 7(A) or the light emitting element 7(B) from the exterior of the mouthpiece 12. Further, the power feeding coil can be used to charge the chargeable battery. Furthermore, the need to mount the large push switch 10 and button battery 9 on the flexible board 6 is eliminated. As a result, the electrical component 5(A) or 5(B) (abbreviated hereafter to 5(A, B)) can be reduced in size and weight, leading to an improvement in an attachment feeling of the mouthpiece 12.

In the therapeutic dental mouthpiece-forming sheet-form substrate 2(A) according to the first embodiment, the flexible board 6 is embedded in the thermosoftening resin sheet 1 in an airtight or watertight state. The flexible board 6 is embedded in the thermosoftening resin sheet 1 by insert molding. As noted above, EVA is used as the material of the thermosoftening resin sheet 1. Rather than using a commercially available sheet, the thermosoftening resin sheet 1 is formed in sheet form in the size of a commercially available sheet using a molding die of an insert molding apparatus. Note, however, that the thermosoftening resin sheet 1 does not necessarily have to be formed in a sheet form of this size.

In the therapeutic dental mouthpiece-forming sheet-form substrate 2(A) according to the first embodiment, the flexible board 6 mounted with the electrical component 5(A, B) used for treatment is embedded in the thermosoftening resin sheet 1 in an airtight or watertight state. An operation to embed the electrical component 5(A, B) can be performed by a manufacturer or a vendor who is accustomed to handling the electrical component 5(A, B), and therefore safety is assured. Using the mouthpiece-forming sheet-form substrate 2(A), a dentist or the like can form the therapeutic dental mouthpiece 12 at low cost by performing a substantially identical operation to a conventional operation with a commercially available forming apparatus 20 to be described below (see Fig. 4). In the therapeutic dental mouthpiece 12, the electrical component 5(A, B) can be disposed accurately in a predetermined treatment position, and the mouthpiece 12 attached with stability so as to align with (fit) a dental arch 14.

Figs. 3A and 3B show a therapeutic dental mouthpiece-forming sheet-form substrate 2(B) according to a second embodiment, wherein Fig. 3A is an exploded side view showing the therapeutic dental mouthpiece-forming sheet-form substrate 2(B) before embedding the flexible board 6 on which the electrical component 5(A, B) is mounted, and Fig. 3B is a sectional view corresponding to an I-I line in each of Figs. 2A and 2B.

In the therapeutic dental mouthpiece-forming sheet-form substrate 2(B) according to the second embodiment, the flexible board 6 is sandwiched between two commercially available thermosoftening resin sheets 1 in an airtight or watertight state. In other words, the flexible board 6 is embedded between the two thermosoftening resin sheets 1.

In the therapeutic dental mouthpiece-forming sheet-form substrate 2(B) according to the second embodiment, the flexible board 6 mounted with the electrical component 5(A, B) is sandwiched between the two thermosoftening resin sheets 1 in an airtight or watertight state. As a result, the insert molding apparatus for insert-molding the electrical component 5(A, B) is not required, and therefore the therapeutic dental mouthpiece-forming sheet-form substrate 2(B) can be formed at a lower cost. Further, a dentist or the like who is accustomed to handling the electrical component 5(A, B) can embed the electrical component 5(A, B) together with the flexible board 6 using the commercially available resin sheet 1 and the commercially available forming apparatus 20.

Next, an outline of an operation for manufacturing a therapeutic dental mouthpiece-forming substrate 2(C) according to a third embodiment by preforming the therapeutic dental mouthpiece-forming sheet-form substrate 2(A) or 2(B) according to the first or second embodiment into a steric shape will be described.

Fig. 5B shows the therapeutic dental mouthpiece-forming substrate 2(C) according to the third embodiment preformed into a steric shape, and Fig. 6B shows a therapeutic dental mouthpiece-forming substrate 2(D) according to a fourth embodiment preformed into a steric shape.

Fig. 4 is a perspective view of the commercially available forming apparatus 20. The forming apparatus 20 includes a stage 21 on which a general purpose dental model 13(A) or 13(B) (see Figs. 5A and 6A) is placed. The forming apparatus 20 also includes a main body 23 embedded with a pump or the like that performs suction through a large number of suction holes 22 formed in the stage 21. The forming apparatus 20 further includes an upper-lower pair of sheet fixing tools 25 and 26 that can be raised and lowered freely by struts 24 standing upright on the main body 23 and that sandwich the therapeutic dental mouthpiece-forming substrate 2(A) or 2(B), and an electric heater 27 supported from the struts 24.

Here, the general purpose dental model 13 is a quasi-dental model manufactured to fit any dental arch 14 rather than a precisely extracted dental model manufactured from the dental arch 14 of a user (see Fig. 11A) by a dentist or the like.

As shown in Fig. 5A, for example, the general purpose dental model 13(A) is substantially semicircular and has an arc-shaped (arch-shaped) side face 13a that follows a curve of a front surface 14a of the dental arch 14 when seen from above. Further, as shown in Fig. 6A, the general purpose dental model 13(B) is substantially crescent-shaped so as to follow the curve of the front surface 14a and a curve of a rear surface 14c of the dental arch 14 when seen from above.

One of the general purpose dental models 13(A) and 13(B), for example the general purpose dental model 13(A), is placed on the stage 21 of the forming apparatus 20, as shown in Fig. 4.

Further, a peripheral edge portion of one of the therapeutic dental mouthpiece-forming sheet-form substrates 2(A) and 2(B), for example the therapeutic dental mouthpiece-forming sheet-form substrate 2(A), is sandwiched between the sheet fixing tools 25 and 26.

Next, the therapeutic dental mouthpiece-forming substrate 2(A) is lowered together with the sheet fixing tools 25 and 26 so as to approach the general purpose dental model 13(A). In this state, marks 16 (described below) serving as positioning references are used to make small adjustments to a placing position of the general purpose dental model 13(A), thereby ensuring that the electrical component (5A, B) is positioned in a treatment part of the dental arch 14.

Next, the therapeutic dental mouthpiece-forming substrate 2(A) is raised together with the sheet fixing tools 25 and 26 and heated by the electric heater 27 until softened. When the therapeutic dental mouthpiece-forming substrate 2(A) is softened, the therapeutic dental mouthpiece-forming substrate 2(A) is lowered together with the sheet fixing tools 25 and 26 such that the softened therapeutic dental mouthpiece-forming substrate 2(A) gradually covers the general purpose dental model 13(A). At this time, suction is performed through the suction holes 22 so that the therapeutic dental mouthpiece-forming substrate 2(A) is tightly adhered to the general purpose dental model 13(A) by a corresponding air flow. As a result, the shape of the general purpose dental model 13(A) is imparted to the therapeutic dental mouthpiece-forming substrate 2(A).

By forming the therapeutic dental mouthpiece-forming sheet-form substrate 2(A) in this manner, the therapeutic dental mouthpiece-forming substrate 2(C) according to the third embodiment, preformed into a steric shape, is completed, as shown in Figs. 5B and 5C. This method of forming the therapeutic dental mouthpiece-forming substrate 2(C) is substantially identical to a conventional mouthpiece forming method.

In the therapeutic dental mouthpiece-forming substrate 2(C) according to the third embodiment, the electrical component 5(A, B) on the flexible board 6 is positioned in a treatment part on either the front surface 14a or an occlusion surface 14b (in this example, the front surface 14a) of the dental arch 14 (see Fig. 11 A) of the user. The therapeutic dental mouthpiece-forming substrate 2(C) is preformed into a steric body (an unfinished product) having a front surface portion 2a that extends substantially along the front surface 14a and an occlusion surface portion 2b that extends substantially along the occlusion surface 14b, and a cavity portion 2d serving as a part that corresponds to the rear surface 14c of the dental arch 14.

A part of the therapeutic dental mouthpiece-forming substrate 2(C) according to the third embodiment extends substantially along the front surface 14a and the occlusion surface 14b of the dental arch 14 of the user. Further, the part facing the rear surface 14c is preformed into a steric body constituting the cavity portion 14d. Hence, using the therapeutic dental mouthpiece-forming substrate 2(C) preformed into a steric body, a dentist or the like can form the therapeutic dental mouthpiece 12 at low cost by performing a substantially identical operation to a conventional operation with the commercially available forming apparatus 20. In particular, the electrical component 5(A, B) on the flexible board 6 can be positioned in advance in the treatment part of the dental arch 14 of the user, and therefore the electrical component 5(A, B) can be disposed accurately in a predetermined treatment position.

Further, in the method of preforming the steric therapeutic dental mouthpiece-forming substrate 2(C), the therapeutic dental mouthpiece-forming sheet-form substrate 2(A) or 2(B) is placed over the general purpose dental model 13(A) or 13(B) in a softened state such that the shape of the general purpose dental model is imparted to the therapeutic dental mouthpiece-forming sheet-form substrate 2(A) or 2(B). As a result, the therapeutic dental mouthpiece-forming sheet-form substrate 2(A) or 2(B) can be preformed into the steric therapeutic dental mouthpiece-forming substrate 2(C) easily and at low cost by performing a substantially identical operation to a conventional operation using the commercially available forming apparatus 20.

As shown in Figs. 6B and 6C, the therapeutic dental mouthpiece-forming substrate 2(D) according to the fourth embodiment, preformed into a steric shape, can be manufactured similarly to the third embodiment by placing the general purpose dental model 13(B) on the stage 21 of the forming apparatus 20.

In the therapeutic dental mouthpiece-forming substrate 2(D) according to the fourth embodiment, the electrical component 5(A, B) on the flexible board 6 is positioned in a treatment part on either the front surface 14a or the occlusion surface 14b (in this example, the front surface 14a) of the dental arch 14 (see Fig. 11 A) of the user. The therapeutic dental mouthpiece-forming sheet-form substrate 2(A) or 2(B) is then preformed into a steric body (an unfinished product) in which the front surface portion 2a, the occlusion surface portion 2b, and the rear surface portion 2c extends substantially along the front surface 14a, the occlusion surface 14b, and the rear surface 14c.

The therapeutic dental mouthpiece-forming substrate 2(D) according to the fourth embodiment is preformed into a steric body in which a part of the therapeutic dental mouthpiece-forming substrate 2(C) extends substantially along the front surface 14a, the occlusion surface 14b, and the rear surface 14c of the dental arch 14 of the user. Hence, using the therapeutic dental mouthpiece-forming substrate 2(D) preformed into a steric body, a dentist or the like can form the therapeutic dental mouthpiece 12 at low cost by performing a substantially identical operation to a conventional operation with the commercially available forming apparatus 20. In particular, the electrical component 5(A, B) on the flexible board 6 can be positioned in advance in the treatment part of the dental arch 14 of the user, and therefore the electrical component 5(A, B) can be disposed accurately in the predetermined treatment position.

Next, an outline of an operation for preforming the therapeutic dental mouthpiece-forming substrate 2(C) according to the third embodiment or the therapeutic dental mouthpiece-forming substrate 2(D) according to the fourth embodiment using the therapeutic dental mouthpiece-forming sheet-form substrate 2(B) according to the second embodiment will be described.

One of the general purpose dental models 13(A) and 13(B), for example the general purpose dental model 13(A), is placed on the stage 21 of the forming apparatus 20, as shown in Fig. 4.

The first resin sheet 1 is then sandwiched between the sheet fixing tools 25 and 26, whereupon small adjustments are made to the general purpose dental model 13(A). Next, the first resin sheet 1 is raised together with the sheet fixing tools 25 and 26 and heated by the electric heater 27 until softened. When the first resin sheet 1 is softened, the first resin sheet 1 is lowered together with the sheet fixing tools 25 and 26 such that the softened first resin sheet 1 gradually covers the general purpose dental model 13(A). At this time, suction is performed through the suction holes 22 such that the first resin sheet 1 is tightly adhered to the general purpose dental model 13(A) by a corresponding air flow. As a result, the shape of the general purpose dental model 13(A) is imparted to the first resin sheet 1.

Next, while the shaped first resin sheet 1 remains hot, the flexible board 6 is adhered to the first resin sheet 1 such that the electrical component 5(A, B) is positioned in the treatment part of the dental arch.

Further, the second resin sheet 1 is placed over the first resin sheet 1 adhered with the flexible board 6 using the sheet fixing tools 25 and 26 in a similar manner to that described above. At this time, suction is performed through the suction holes 22 such that the second resin sheet 1 is tightly adhered to the first resin sheet 1 by a corresponding air flow. As a result, the shape of the first resin sheet 1 is imparted to the second resin sheet. Thus, using the two resin sheets 1, the flexible board 6 is embedded between the two resin sheets 1 in an airtight or watertight state.

In this preforming method, the first resin sheet 1 is placed over the general purpose dental model 13(A) in a softened state such that the shape of the general purpose dental model 13(A) is imparted to the first resin sheet 1. The flexible board 6 is then adhered to the first resin sheet 1 such that the electrical component 5(A, B) is positioned in the treatment part. Next, the second resin sheet 1 is placed over the first resin sheet 1 in a softened state such that the shape of the first resin sheet 1 is imparted to the second resin sheet 1. As a result, the steric therapeutic dental mouthpiece-forming substrate 2(C) or 2(D) can be preformed easily and at low cost by performing a substantially identical operation to a conventional operation using the commercially available forming apparatus 20.

When the therapeutic dental mouthpiece-forming sheet-form substrate 2(C) or 2(D) is preformed using the therapeutic dental mouthpiece-forming sheet-form substrate 2 (2(A) or 2(B)) shown in Figs. 2 and 3, positioning marks relative to the dental arch 14 are preferably provided on the resin sheet 1.

As shown in Fig. 7A (see also Figs. 2, 5, and 6), for example, marks 16 are formed in advance on the therapeutic dental mouthpiece-forming sheet-form substrate 2 in the form of lines (straight lines, broken lines, or the like), ridges, or the like extending radially at equal angular intervals (45°, for example) from a center O. Further, as shown in Fig. 7B, hook-shaped marks 16 or the like indicating the position of the flexible board 6 embedded in the substrate 2 are formed in advance on the therapeutic dental mouthpiece-forming sheet-form substrate 2.

The marks 16 can be used as references for positioning the electrical component 5(5(A) or 5(B)) in the treatment part of the dental arch 14 during the operation for preforming the steric therapeutic dental mouthpiece-forming substrate 2 (2(C) or 2(D)) from the sheet-form mouthpiece-forming substrate 2. As a result, the operation for positioning the electrical component 5 in the treatment part can be performed easily.

Further, the marks 16 are preferably embedded in the resin sheet 1. More specifically, the mouthpiece 12 is used inside the oral cavity, and therefore biostability must be secured therein. Since the marks 16 are embedded in the resin sheet 1, however, the marks 16 themselves do not necessarily have to exhibit stability inside the oral cavity.

The therapeutic dental mouthpiece-forming substrates 2(A) to 2(D) described above must be formed into the shape of the finished product mouthpiece 12 that fits the dental arch 14 of the user in a main forming operation. The commercially available forming apparatus 20 shown in Fig. 4 can be used in the main forming operation (ultimate forming operation).

First, a method of forming the finished product mouthpiece 12 using the therapeutic dental mouthpiece-forming sheet-form substrates 2(A) and 2(B) will be described.

An extracted dental model 33 of the user (see Fig. 8) is manufactured by attaching an impression material to the dental arch 14 of the user in a dental clinic to model the dental arch 14, casting gypsum into the impression material carrying the shape of the dental arch in a dental studio, and then hardening and removing the gypsum.

Next, as shown in Fig. 8, the extracted dental model 33 of the user is placed on the stage 21 of the forming apparatus 20. Further, the peripheral edge portion of one of the therapeutic dental mouthpiece-forming sheet-form substrates 2(A) and 2(B), for example the therapeutic dental mouthpiece-forming sheet-form substrate 2(A), is sandwiched between the sheet fixing tools 25 and 26.

Next, the therapeutic dental mouthpiece-forming substrate 2(A) is lowered together with the sheet fixing tools 25 and 26 so as to approach the extracted dental model 33. In this state, the marks 16 serving as positioning references are used to make small adjustments to the placing position of the extracted dental model 33, thereby ensuring that the electrical component 5(A) or 5(B) is positioned in the treatment part of the dental arch.

Next, the therapeutic dental mouthpiece-forming substrate 2(A) is raised together with the sheet fixing tools 25 and 26 and heated by the electric heater 27 until softened. When the therapeutic dental mouthpiece-forming substrate 2(A) is softened, the therapeutic dental mouthpiece-forming substrate 2(A) is lowered together with the sheet fixing tools 25 and 26 such that the softened therapeutic dental mouthpiece-forming substrate 2(A) gradually covers the extracted dental model 33. At this time, suction is performed through the suction holes 22 so that the therapeutic dental mouthpiece-forming substrate 2(A) is tightly adhered to the extracted dental model 33 by a corresponding air flow.

After performing the main forming operation in this manner, the dentist or the like cuts away surplus parts such as flange parts using a cutter or the like once these parts have hardened, whereby the mouthpiece 12 serving as the finished product is formed as shown in Fig. 11B.
This method of forming the mouthpiece 12 is substantially identical to a conventional mouthpiece forming method.

A method of forming the mouthpiece 12 serving as the finished product using the therapeutic dental mouthpiece-forming substrate 2(C) or 2(D) preformed into a steric shape is similar to this method, and therefore detailed description thereof has been omitted.

Hence, the dentist or the like can form the mouthpiece 12 by performing a substantially identical operation to a conventional operation using the commercially available forming apparatus 20. In this operation, the electrical component 5(A) or 5(B) is disposed accurately in the predetermined treatment position. The therapeutic dental mouthpiece 12 obtained as a result is aligned with (fits) the dental arch 14, and therefore exhibits superior stability when attached.

Alternatively, using the commercially available forming apparatus 20 shown in Fig. 4, the dentist or the like can form the mouthpiece 12 serving as the finished product from the sheet-form mouthpiece-forming substrate 2(B) directly, i.e. without preforming the sheet-form mouthpiece-forming substrate 2(B) into a steric shape. More specifically, in a forming operation performed in this case, as shown in Fig. 8, the extracted dental model 33 of the user is placed on the stage 21 of the forming apparatus 20. The first resin sheet 1 is then sandwiched between the sheet fixing tools 25 and 26, whereupon small adjustments are made to the extracted dental model 33. Next, the first resin sheet 1 is raised together with the sheet fixing tools 25 and 26 and heated by the electric heater 27 until softened.

When the first resin sheet 1 is softened, the first resin sheet 1 is lowered together with the sheet fixing tools 25 and 26 such that the softened first resin sheet 1 gradually covers the extracted dental model 33. At this time, suction is performed through the suction holes 22 so that the first resin sheet 1 is tightly adhered to the extracted dental model 33 by a corresponding air flow. Accordingly, the shape of the extracted dental model 33 is imparted to the first resin sheet 1. As a result of this forming operation, as shown in Fig. 10A, the first resin sheet 1 is formed into the inside piece 12A taking the shape of the extracted dental model 33.

Next, as shown in Fig. 10B, while the inside piece 12A remains hot, the flexible board 6 is adhered to the inside piece 12A such that the electrical component 5(A) or 5(B) is positioned in the treatment part of the dental arch.

Further, the second resin sheet 1 is placed over the inside piece 12A adhered with the flexible board 6 using the sheet fixing tools 25 and 26 in a similar manner to that described above. At this time, suction is performed through the suction holes 22 so that the second resin sheet 1 is tightly adhered to the inside piece 12A by a corresponding air flow. As a result, as shown in Fig. 10C, the shape of the inside piece 12A is imparted to the second resin sheet, thereby completing an outside piece 12B.

The flexible board 6 is embedded in an airtight or watertight state by the inside piece (the first resin sheet 1) 12A and the outside piece (the second resin sheet 1) 12B. The mouthpiece 12 serving as the finished product is then formed by cutting away surplus parts such as flange parts once these parts have hardened, as described above.

Hence, the dentist or the like can form the mouthpiece 12 by performing a substantially identical operation to a conventional operation using the commercially available forming apparatus 20. In this operation, the electrical component 5(A) or 5(B) is disposed accurately in the predetermined treatment position. The therapeutic dental mouthpiece 12 obtained as a result is aligned with (fits) the dental arch 14, and therefore exhibits superior stability when attached.

Next, an outline of an operation with which the user him/herself can form the mouthpiece 12 easily using the therapeutic dental mouthpiece-forming substrate 2(A) according to the first embodiment or the therapeutic dental mouthpiece-forming substrate 2(B) according to the second embodiment will be described.

As shown in Fig. 9A, in the sheet-form mouthpiece-forming substrate 2(A) or 2(B), the electrical component 5(A) or 5(B) on the flexible board 6 is positioned in the treatment part on either the front surface 14a or the occlusion surface 14b of the dental arch 14 of the user.

The sheet-form mouthpiece-forming substrate 2(A) or 2(B) is then cut using a cutter or the like to obtain a substrate piece 2'. As will be described below, the substrate piece 2' can be formed in a steric shape that substantially aligns with the front surface 14a, the occlusion surface 14b, and the rear surface 14c. In an developed state before being formed into a steric shape, the substrate piece 2' is quadrangular. Note that the substrate piece 2' is not limited to a horizontally long quadrangular shape, and may be formed in an elliptical shape or the like.

The softened substrate piece 2' is then placed over the general purpose dental model 13(B) shown in Fig. 9B such that the vibrator 7(A) or the light emitting element 7(B) constituting the electrical component 5 on the flexible board 6 is positioned in the treatment part of the dental arch 14. As a result, the shape of the general purpose dental model 13(B) is imparted to the substrate piece 2', as shown in Figs. 9C and 9D.

The steric body preformed in this manner has an arc shape (an arch shape) that follows the curve of the dental arch when seen from above and a U shape that fits over the dental arch 14 when seen from the side. In other words, the steric body has a steric shape that curves so as to be substantially in alignment with the front surface 14a, the occlusion surface 14b, and the rear surface 14c of the dental arch.

The substrate piece 2' is quadrangular in the developed state before being preformed into the steric shape that substantially aligns with the front surface 14a, the occlusion surface 14b, and the rear surface 14c of the dental arch 14 of the user, and therefore substrate piece 2' has a small shape.

The substrate piece 2' is preformed into the steric body that curves so as to be substantially in alignment with the front surface 14a, the occlusion surface 14b, and the rear surface 14c of the dental arch 14 using the general purpose dental model 13(B). The user then attaches the substrate piece 2' to the dental arch 14 after softening the substrate piece 2' in warm water, for example. In this state, the user can form the final (finished) therapeutic dental mouthpiece 12 easily by closing his/her upper and lower dental arches 14 or the like. In the mouthpiece 12, the electrical component 5(A) or 5(B) is disposed accurately in the predetermined treatment position. Further, the mouthpiece 12 is aligned with (fits) the dental arch 14 and therefore exhibits superior stability when attached.

In the embodiments described above, the general purpose dental model 13(A) or 13(B) is substantially arc-shaped (arch-shaped) or crescent-shaped so as to follow the curve of the dental arch when seen from above. Taking personal differences into consideration, however, the general purpose dental model 13(A) or 13(B) is preferably prepared in two sizes (large and small) to fit an adult and a child or three sizes (large, medium, and small) to fit an adult man, an adult woman, and a child, for example.

Further, dental arches can be roughly divided into four types, namely a substantially U-shaped type, a substantially V-shaped type, a substantially square type, and a substantially saddle-shaped type. Of these types, almost eighty percent of people have substantially U-shaped dental arches. Therefore, by arranging the electrical components 5 in a substantially U-shaped pattern, the mouthpiece can be made compatible with almost all dental arch shapes.

Further, of the four dental arch shapes, the substantially U-shaped type, the substantially square type, and the substantially saddle-shaped type do not differ greatly from each other, and therefore the substantially U-shaped type can be formed into the substantially square type or the substantially saddle-shaped type by making adjustments thereto during the final forming operation. However, the substantially V-shaped type differs greatly from the other three dental arch shapes in that a front tooth part takes a pointed shape. Hence, a mouthpiece in which the electrical components 5 are arranged in a substantially V-shaped pattern is preferably also prepared.

### (Summary of the Embodiments)

The embodiments described above can be summarized as follows.

(1) The therapeutic dental mouthpiece-forming substrate according to the above embodiments includes: a flexible board on which an electrical component used for dental arch treatment is mounted; and a thermosoftening resin sheet in which the flexible board is embedded in an airtight or watertight state.

In the therapeutic dental mouthpiece-forming sheet-form substrate according to this aspect, the flexible board mounted with the electrical component used for treatment is embedded in the thermosoftening resin sheet in an airtight or watertight state. The operation to embed the electrical component can be performed by a manufacturer or a vendor who is accustomed to handling the electrical component, and therefore safety is assured. Using this mouthpiece-forming substrate, a dentist or the like can form a therapeutic dental mouthpiece at low cost by performing a substantially identical operation to a conventional operation with a commercially available forming apparatus. In the therapeutic dental mouthpiece obtained in this manner, the electrical component is disposed accurately in a predetermined treatment position, and the mouthpiece aligns with (fits) the dental arch. As a result, the therapeutic dental mouthpiece exhibits superior stability when attached.

(2) As described above in the second embodiment, the flexible board mounted with the electrical component may be embedded in the thermosoftening resin sheet by being sandwiched between two resin sheets in an airtight or watertight state.

According to this aspect, the flexible board mounted with the electrical component can be embedded in the thermosoftening resin sheet by being sandwiched between the two resin sheets in an airtight or watertight state, and therefore an insert molding apparatus for insert-molding the electrical component is not required. As a result, the therapeutic dental mouthpiece-forming substrate can be formed at a lower cost. Further, a dentist or the like who is accustomed to handling the electrical component can embed the electrical component together with the flexible board using commercially available resin sheets and a commercially available forming apparatus.

(3) As described above in the third embodiment, the therapeutic dental mouthpiece-forming sheet-form substrate according to the first or second embodiment may be preformed into a steric body. In the steric body, a part of the therapeutic dental mouthpiece-forming sheet-form substrate extends substantially along at least the front surface and the occlusion surface of the dental arch of the user, thereby ensuring that the electrical component on the flexible board is positioned in a treatment part on either the front surface or the occlusion surface, while a cavity portion is formed in the part facing the rear surface of the dental arch.

Using the therapeutic dental mouthpiece-forming substrate preformed into a steric shape according to this aspect, a dentist or the like can form a therapeutic dental mouthpiece that can be attached with stability so as to align with (fit) the dental arch at low cost by performing a substantially identical operation to a conventional operation with a commercially available forming apparatus. In particular, the electrical component on the flexible board can be positioned in advance in the treatment part of the dental arch of the user, and therefore the electrical component can be disposed accurately in a predetermined treatment position.

(4) As described above in the fourth embodiment, the therapeutic dental mouthpiece-forming sheet-form substrate according to the first or second embodiment may be preformed into a steric body. In the steric body, a part of the therapeutic dental mouthpiece-forming sheet-form substrate expands substantially along the front surface, the occlusion surface, and the rear surface of the dental arch of the user, thereby ensuring that the electrical component on the flexible board is positioned in a treatment part on either the front surface or the occlusion surface.

Using the therapeutic dental mouthpiece-forming substrate preformed into a steric shape according to this aspect, a dentist or the like can form a therapeutic dental mouthpiece that can be attached with stability so as to align with (fit) the dental arch at low cost by performing a substantially identical operation to a conventional operation with a commercially available forming apparatus. In particular, the electrical component on the flexible board can be positioned in advance in the treatment part of the dental arch of the user, and therefore the electrical component can be disposed accurately in a predetermined treatment position.

(5) In the therapeutic dental mouthpiece-forming substrate described above, a positioning mark relative to the dental arch is preferably formed on the resin sheet.

According to this aspect, a line (a straight line, a broken line, or the like), a ridge, or the like, for example, is formed as the mark on the resin sheet. The formed mark serves as a positioning reference for positioning the electrical component in the treatment part of the dental arch during the operation for preforming the mouthpiece-forming substrate into a steric shape, and therefore the electrical component can be positioned easily.

(6) In the therapeutic dental mouthpiece-forming substrate described above, the mark is preferably embedded in the resin sheet.

The mouthpiece is used inside the oral cavity, and therefore biostability must be secured therein. According to this aspect, however, the mark is embedded in the resin sheet, and therefore the mark itself (the material of the mark) does not necessarily have to be assured for its safety for the use inside the oral cavity.

(7) In the therapeutic dental mouthpiece-forming substrate described above, the electrical component, when used to perform whitening treatment on the dental arch, preferably includes a light emitting element for performing whitening treatment on the dental arch, a control element for controlling the light emitting element, a power supply battery, and a power supply switch. Further, when used to perform orthodontic treatment on the dental arch, the electrical component preferably includes a vibrator for performing orthodontic treatment on the dental arch, a control element for controlling the vibrator, a power supply battery, and a power supply switch.

(8) A method of preforming the therapeutic dental mouthpiece-forming sheet-form substrate according to the first or second embodiment into the steric therapeutic dental mouthpiece-forming substrate according to the third or fourth embodiment includes: a step of positioning the therapeutic dental mouthpiece-forming sheet-form substrate relative to a general purpose dental model so that the electrical component on the flexible board is positioned in the treatment part; and a step of suctioning the therapeutic dental mouthpiece-forming sheet-form substrate in a softened state, with the general purpose dental model being covered with the therapeutic dental mouthpiece-forming sheet-form substrate, whereby a shape of the general purpose dental model is imparted to the therapeutic dental mouthpiece-forming sheet-form substrate.

Using the therapeutic dental mouthpiece-forming substrate preformed into a steric shape according to this aspect, a dentist or the like can form a therapeutic dental mouthpiece in which the electrical component is disposed accurately in a predetermined treatment position and which can be attached with stability so as to align with (fit) the dental arch at low cost by performing a substantially identical operation to a conventional operation with a commercially available forming apparatus.

Here, the general purpose dental model is a quasi-dental model manufactured to fit any dental arch rather than a precisely extracted dental model manufactured from the dental arch of a user by a dentist or the like.

(9) A method of preforming two resin sheets and an electrical component into the steric therapeutic dental mouthpiece-forming substrate according to the third or fourth embodiment includes: a step of suctioning a first resin sheet in a softened state, with a general purpose dental model being covered with the first resin sheet, whereby a shape of the general purpose dental model is imparted to the first resin sheet; a step of attaching the flexible board to the shaped first resin sheet so that the electrical component on the flexible board is positioned in the treatment part; and a step of embedding the flexible board between the two resin sheets by suctioning a second resin sheet in a softened state, with the first resin sheet being covered with the second resin sheet, whereby a shape of the first resin sheet is imparted to the second resin sheet and the flexible board is sandwiched between the two resin sheets in an airtight or watertight state.

Using the therapeutic dental mouthpiece-forming substrate preformed into a steric shape according to this aspect, a dentist or the like can form a therapeutic dental mouthpiece in which the electrical component is disposed accurately in a predetermined treatment position and which can be attached with stability so as to align with (fit) the dental arch at low cost by performing a substantially identical operation to a conventional operation with a commercially available forming apparatus.

(10) A method of preforming the therapeutic dental mouthpiece-forming sheet-form substrate according to the first or second embodiment into the steric therapeutic dental mouthpiece-forming substrate according to the fourth embodiment may also be employed. In this preforming method, the therapeutic dental mouthpiece-forming sheet-form substrate is cut into a substrate piece having a developed quadrangular shape that can substantially align with the front surface, the occlusion surface, and the rear surface of the dental arch of the user, thereby ensuring that the electrical component on the flexible board is positioned in the treatment part on either the front surface or the occlusion surface, and the substrate piece is preformed into a steric body by placing the substrate piece over a general purpose dental model in a softened state so as to curve substantially along the front surface, the occlusion surface, and the rear surface.

According to this aspect, the substrate piece expands into a quadrangular shape that substantially aligns with the front surface, the occlusion surface, and the rear surface of the dental arch of the user, and therefore the substrate piece has a small shape. Using the general purpose dental model, the substrate piece is preformed into a steric body that curves substantially along the front surface, the occlusion surface, and the rear surface of the dental arch. The user places the substrate piece to his/her dental arch after softening the substrate piece in warm water, for example, and then performs an operation such as closing his/her upper and lower dental arches. As a result, a final (finished) therapeutic dental mouthpiece in which the electrical component is disposed accurately in a predetermined treatment position and which can be attached with stability so as to align with (fit) the dental arch can be formed easily.

### Explanation of Reference Numerals

1 thermosoftening resin sheet
2(A) to 2(D) therapeutic dental mouthpiece-forming substrate
2' substrate piece
5(A) and 5(B) electrical component
6 flexible board
7(A) vibrator
7(B) light emitting element
8 control element
9 power supply battery
10 power supply switch
12 mouthpiece
13(A) and 13(B) general purpose dental model
14 dental arch
14a front surface
14b occlusion surface
14c rear surface
14d cavity portion
16 mark
20 forming apparatus

## Claims

1. A therapeutic dental mouthpiece-forming substrate comprising:
a flexible board on which an electrical component used for dental treatment is mounted; and
a thermosoftening resin sheet in which the flexible board is embedded in an airtight or watertight state.

2. The therapeutic dental mouthpiece-forming substrate according to claim 1, wherein
the thermosoftening resin sheet includes two resin sheets, and
the flexible board mounted with the electrical component is embedded in the thermosoftening resin sheet by being sandwiched between the two resin sheets in an airtight or watertight state.

3. The therapeutic dental mouthpiece-forming substrate according to claim 1 or 2, wherein
the therapeutic dental mouthpiece-forming substrate is preformed into a steric body, and
in the steric body, a part of the therapeutic dental mouthpiece-forming substrate extends substantially along at least a front surface and an occlusion surface of a dental arch of a user so that the electrical component on the flexible board is positioned in a treatment part on either the front surface or the occlusion surface, while a cavity portion is formed in a part facing a rear surface of the dental arch.

4. The therapeutic dental mouthpiece-forming substrate according to claim 1 or 2, wherein
the therapeutic dental mouthpiece-forming substrate is preformed into a steric body, and
in the steric body, a part of the therapeutic dental mouthpiece-forming substrate extends substantially along a front surface, an occlusion surface, and a rear surface of a dental arch of a user so that the electrical component on the flexible board is positioned in a treatment part on either the front surface or the occlusion surface.

5. The therapeutic dental mouthpiece-forming substrate according to any one of claims 1 to 4, wherein a positioning mark relative to the dental arch is formed on the thermosoftening resin sheet.

6. The therapeutic dental mouthpiece-forming substrate according to claim 5, wherein the mark is embedded in the thermosoftening resin sheet.

7. The therapeutic dental mouthpiece-forming substrate according to any one of claims 1 to 6, wherein
the electrical component comprises:
a light emitting element for performing whitening treatment on the dental arch or a vibrator for performing orthodontic treatment on the dental arch;
a control element for controlling the light emitting element or the vibrator;
a power supply battery; and
a power supply switch.

8. A preforming method for a therapeutic dental mouthpiece-forming substrate, the method being used to preform a therapeutic dental mouthpiece-forming sheet-form substrate into the therapeutic dental mouthpiece-forming substrate according to claim 3 or 4, and comprising:
a step of positioning the therapeutic dental mouthpiece-forming sheet-form substrate relative to a general purpose dental model so that the electrical component on the flexible board is positioned in the treatment part; and
a step of suctioning the therapeutic dental mouthpiece-forming sheet-form substrate in a softened state, with the general purpose dental model being covered with the therapeutic dental mouthpiece-forming sheet-form substrate, whereby a shape of the general purpose dental model is imparted to the therapeutic dental mouthpiece-forming sheet-form substrate.

9. A preforming method for a therapeutic dental mouthpiece-forming substrate, the method being used to preform two resin sheets and an electrical component into the therapeutic dental mouthpiece-forming substrate according to claim 3 or 4, and comprising:
a step of suctioning a first resin sheet in a softened state, with a general purpose dental model being covered with the first resin sheet, whereby a shape of the general purpose dental model is imparted to the first resin sheet;
a step of attaching the flexible board to the shaped first resin sheet so that the electrical component on the flexible board is positioned in the treatment part; and
a step of embedding the flexible board between the two resin sheets by suctioning a second resin sheet in a softened state, with the first resin sheet being covered with the second resin sheet, whereby a shape of the first resin sheet is imparted to the second resin sheet and the flexible board is sandwiched between the two resin sheets in an airtight or watertight state.

10. A preforming method for a therapeutic dental mouthpiece-forming substrate, the method being used to preform a therapeutic dental mouthpiece-forming sheet-form substrate into the therapeutic dental mouthpiece-forming substrate according to claim 4, wherein
the therapeutic dental mouthpiece-forming sheet-form substrate is cut into a substrate piece having a developed quadrangular shape that can substantially align with the front surface, the occlusion surface, and the rear surface of the dental arch of the user, thereby ensuring that the electrical component on the flexible board is positioned in the treatment part on either the front surface or the occlusion surface, and
the substrate piece is preformed into a steric body by placing the substrate piece over a general purpose dental model in a softened state so as to curve substantially along the front surface, the occlusion surface, and the rear surface.
